# EUROPEAN PATENT APPLICATION

(11) **EP 4 778 505 A1**
(43) Date of publication of application: **22.07.2026**
(21) Application number: 24876355.9
(22) Date of filing: 19.09.2024
(51) Int. Cl.: A61K 8/02, A61Q 19/00

(54) **PROCESS METHOD FOR IMPROVING QUALITY AND STABILITY OF POWDER PRODUCT AND USE THEREOF**

(30) Priority: 10.10.2023 CN 202311310026
(71) Applicant: A & H International Cosmetics Co., Ltd, Shanghai 201415 (CN)
(72) Inventor: TIAN, Yuncai, Shanghai 201415 (CN); TIAN, Yong, Shanghai 201415 (CN); ZHANG, Jinnv, Shanghai 201415 (CN); WU, Yidi, Shanghai 201415 (CN); CHEN, Jinhai, Shanghai 201415 (CN); SHEN, Jie, Shanghai 201415 (CN)
(74) Representative: Boult Wade Tennant LLP
(86) International application number: PCT/CN2024/119626
(87) International publication number: WO 2025/077536

(57) **Abstract**

A process method for improving the quality and stability of a powder product, at least comprising: preheating a mold, filling, vibrating at the bottom of a mesh, cooling, microwave drying, pre-freezing, demolding, freezing, sublimation drying, and baking. The prepared powder product has an exquisite appearance and is suitable for production and manufacturing of powder cosmetics.

## Description

### CROSS REFERENCE TO RELATED APPLICATION

The present application claims priority to Chinese Patent Application No. 202311310026.1, entitled "PROCESS METHOD FOR IMPROVING QUALITY AND STABILITY OF POWDER PRODUCT AND USE THEREOF" filed on October 10, 2023, the entire contents of which are incorporated herein by reference.

### TECHNICAL FIELD

The present application belongs to the field of cosmetic manufacturing, and particularly relates to A61K8/02, and more specifically to a process method for improving quality and stability of powder product and use thereof.

### BACKGROUND

Powder cosmetics have long been popular among women seeking beauty since ancient times. Common powder cosmetics include products such as eyeshadow, compact powder, blush, contouring powders and the like, which serve to set makeup and conceal imperfections, absorb excess facial oil, reduce facial shine, and prolong the wear of base makeup. They also function to adjust skin tone, making facial makeup softer and more delicate. Typically, powder cosmetics with an attractive appearance, no significant color difference, and a uniform texture are more likely to stimulate consumer purchasing intention. The raw materials for preparing powder cosmetics generally include an oil phase, an emulsifier, a humectant, an aqueous solution, and the like. Usually, producing a powder product requires prolonged drying. However, simply using prolonged drying treatment can easily cause some functional components to volatilize due to heat, and also lead to deformation or shrinkage of the powder cosmetic. Another common method for preparing powder cosmetics is pressing, where powdered solid particles are compressed under a certain pressure. However, this treatment process can lead to a certain decrease in the skin adhesion of the cosmetic, and makeup fallout may occur after use, resulting in a poor user experience.

Prior art CN114504510A discloses a method for manufacturing a freeze-dried cosmetic composition. The mixed raw materials include a water-soluble gelling agent-containing aqueous-phase part, an emulsifier, an oil-phase part, and a powder part, which are then subjected to cooling and freeze-drying steps. The obtained product has an exquisite appearance and a relatively uniform texture. However, it can be seen from product images that the finished product exhibits edge detachment. Prior art CN116139023A discloses a method for preparing powder products, mainly including mixing raw materials, filling, baking, cooling, demolding, and vacuum freeze-drying. The obtained product has stable mechanical properties and qualified moisture content, providing a certain moisturizing effect. However, it is difficult for the above methods to ensure that the powder cosmetics simultaneously possess the advantages of intact edges, low color difference, absence of pores, and no wrinkles.

Therefore, providing a process method for improving the quality and stability of powdery products, enabling the obtained powder products to have an exquisite appearance, uniform texture, intact edges, low color difference, no pores, and no wrinkles, holds significant practical importance.

### SUMMARY

To solve the above problems, a first aspect of the present application provides a process method for improving the quality and stability of powder product, at least comprising the following steps:
S1, Preparation stage: preparing and placing molds;
S2, Preheating a mold: placing the mold in a heating device to preheat the mold;
S3, Filling: placing the material obtained from step S2 into a quantitative filling machine for quantitative filling. According to product weight requirements, the material is quantitatively filled using the quantitative filling machine, with an accuracy of ≤±0.3g;
S4, Bottom vibration with a grid: placing the material obtained from step S3 onto a grid and then placing it in a vibrator, vibrating at a certain frequency;
S5, Cooling: cooling the material obtained from step S4 under low-temperature conditions;
S6, Microwave drying: subjecting the material obtained from step S5 to microwave drying;
S7, Pre-freezing: pre-freezing the material obtained from step S6;
S8, Demolding: demolding the material obtained from step S7;
S9, Freezing: placing the material obtained from step S8 into a freeze dryer for freezing and shaping;
S10, Sublimation drying: placing the material obtained from step S9 into a freeze dryer for stepped sublimation drying;
S11, Baking: heating the material obtained from step S10 by baking;
S12, Low-temperature storage: storing the material obtained from step S11 in a constant temperature and humidity environment.

Preferably, the temperature for preheating the mold in step S2 is 50-350°C; as an implementable example, the temperature for preheating the mold may comprise one of 50°C, 100°C, 150°C, 200°C, 250°C, 300°C, or 350°C.

During experimentation, the inventors have found that preheating the mold before filling can improve issues of large color difference and pores in powdery products. Particularly when the heating temperature is limited to 55-85°C, the occurrence of pores in the prepared powder product can be avoided. In the process of preparing powder products, stages such as filling and drying are generally involved. Temperature increases due to subsequent drying can affect the uniformity of the powder, leading to pore formation. During the investigation process, the inventors examined each process step and ultimately found that preheating the mold before filling the powder product can significantly reduce pore problems in the obtained product and improve the yield rate. The inventors speculate that the possible reason is as follows: the mold preheating process reduces temperature variation, preventing the internal molecules of the material from intensifying irregular motion due to temperature differences within the system, thereby reducing color differences caused by structural changes in the powder product. At the same time, reducing the temperature difference also prevents the material from cooling too quickly during the filling process, allowing the material to flow into every corner and avoiding pore formation. Meanwhile, the inventors further found that excessively high mold preheating temperatures could also damage the raw material system, affecting product yield or surface color.

Preferably, the filling temperature in step S3 is 50-100°C; as an implementable example, the filling temperature may comprise one of 50°C, 60°C, 70°C, 80°C, 90°C, or 100°C.

Preferably, the vibration frequency in step S4 is 100-500 Hz; as an implementable example, the vibration frequency comprises one of 100 Hz, 150 Hz, 200 Hz, 250 Hz, 300 Hz, 400 Hz, 450 Hz, or 500 Hz.

In the present application, a grid needs to be added when filling powder products, and bottom vibration is adopted, but the vibration frequency must be maintained at 100-500 Hz. During the preparation of powder products, traditional methods generally involve mixing raw materials to obtain a water-in-oil/oil-in-water system, followed by filling, cooling, and then baking. Such process methods are simpler, but the resulting products are prone to issues like incomplete edge filling or pore formation. Under the premise of preheating mold, the inventors discovered through investigation that adding bottom vibration and a grid during filling can further alleviate pore formation and ensure regular shape after filling. The applicants speculate that the reason for this phenomenon is: within such range, it ensures no "accumulation" or particle stratification in the material, improves material fluidity, and achieves sufficient filling in the mold. However, if the vibration frequency is too high, it can increase the movement frequency of material particles, leading to particle stratification, and excessive movement frequency may also increase the "floating" degree of the material, reducing the filling effect at the edges. When the vibration frequency is below 100 Hz, the inventors found that its auxiliary effect on the material is insufficient, affecting flatness, and leading to incomplete edge filling.

Preferably, the equipment used in step S5 comprises one of a freezing tunnel, a freezing hood, or a freezer. The low-temperature environment is from -10°C to 25°C, and the cooling time is 2-30 min; as an implementable example, the low temperature may comprise one of -10°C, -5°C, 0°C, 5°C, 15°C, or 25°C; the cooling time may comprise one of 3 min, 4 min, 5 min, 6 min, 7 min, 8 min, 9 min, 10 min, 12 min, 15 min, 20 min, 25 min, or 30 min.

Preferably, step S6 is microwave drying; preferably, the conditions for microwave drying are as follows: total microwave power 25 KW: 15-50% duty cycle, microwave frequency: 300 MHz - 300 GHz, electromagnetic wave wavelength: 1 meter to 1 millimeter, line speed: 0.5-2 m/min, temperature inside the tunnel chamber: 25-65°C.

During experimentation, the inventors found that due to the liquid-phase substances present in the raw materials, surface abnormalities such as pores or wrinkles may appear in the finished powder product. The inventors optimized the preparation process and proposed a microwave drying step. The inventors found that through the microwave drying step, especially under the following process parameters: total microwave power 25 KW: 15-50% duty cycle, microwave frequency: 300 MHz - 300 GHz, electromagnetic wave wavelength: 1 meter to 1 millimeter, line speed: 0.5-2 m/min, and temperature inside the tunnel chamber: 25-65°C, it is possible to reduce the moisture content of the raw materials while avoiding a decline in the performance of active substances in the powder product. However, in this system, the inventors found that products not subjected to microwave drying/baking exhibited a soft texture and powder flying during application. After extensive experimentation, the inventors discovered that adding a microwave drying/baking step before freezing significantly improves the soft texture issue of the powder product. The applicants speculate that the reason for this phenomenon is: in this system, gradual heating ensures stable frequency of intermolecular thermal motion, guaranteeing a relatively strong orderliness in the intermolecular forces and entanglement degree between molecular chains of raw materials such as carrageenan, avoiding the problem of molecular conformations being unable to immediately restore to equilibrium conformations adaptable to environmental conditions due to subsequent low-temperature (-5°C) storage and the occurrence of wrinkles. Moreover, baking before storage reduces the internal moisture content of the raw materials to below 5%, preventing caking issues of the powder product during use, improving the yield rate, and enhancing user satisfaction with the product.

Preferably, the pre-freezing equipment in step S7 comprises one of a liquid nitrogen tunnel, a freezing tunnel, or a freeze dryer; the pre-freezing temperature is from -10°C to -70°C, and the pre-freezing time is 0.1-2 h. As an implementable example, the freeze-drying temperature may comprise one of -10°C, -15°C, -20°C, -35°C, -45°C, -50°C, -60°C, or -70°C; the freeze-drying time comprises one of 0.1 h, 0.3 h, 0.5 h, 1 h, 1.2 h, 1.5 h, or 2 h.

Preferably, the stepped heating in step S10 is performed in increments of 5-15°C per step, with each step lasting 1-4 h, for a total duration of 12-24 h.

Preferably, the stepped heating in step S10 is carried out under negative pressure conditions, with a negative pressure of -1 MPa to 0.4 MPa. As an implementable example, the negative pressure may comprise one of -1 MPa, -0.5 MPa, 0.05 MPa, 0.1 MPa, 0.15 MPa, 0.2 MPa, 0.3 MPa, or 0.4 MPa.

As an implementable example, the time for each step may comprise one of 1 h, 1.5 h, 2 h, 2.5 h, 3 h, 3.5 h, or 4 h; the uniform heating per step comprise one of 5°C, 6°C, 7°C, 8°C, 9°C, 10°C, 11°C, 12°C, 13°C, 14°C, or 15°C.

Further preferably, each step heats by 10°C; each step lasts 2 h, for a total duration of 12 h.

Preferably, the equipment for baking in S11 is a drying room or a constant-temperature oven. The baking conditions are as follows: temperature of 45-65°C; time of 5-10 h. As an implementable example, the heating temperature may comprise one of 45°C, 50°C, 55°C, or 60°C; the time may comprise one of 5 h, 6 h, 7 h, 8 h, 9 h, or 10 h.

A second aspect of the present application provides use of the process method for improving the quality and stability of powder product, which is mainly applied in the preparation of powder cosmetics.

Preferably, the raw materials for preparing the powder cosmetics comprise an oil phase, an aqueous phase, and a powder phase; wherein the aqueous phase comprises one or more of a solvent, a thickener, an emulsifier, and a preservative; the oil phase comprises one or more of a humectant, an emulsifier, a thickener, a fragrance, and a functional component; the powder phase comprises one or more of a filler, a humectant, an antioxidant, and a fragrance.

In some preferred embodiments, the powder product comprises, but is not limited to, a combination of a powder cream and a powder product, a powder cream, or a powder product.

### BENEFICIAL EFFECTS

(1) The present application provides a process method for improving the quality and stability of powder product, which is capable of producing powder cosmetics with uniform texture, intact edges, low color difference, no pores, and no wrinkles, possessing an exquisite appearance, particularly suitable for the production and manufacture of powder cosmetics.
(2) By preheating the mold to a specific temperature, the present application can effectively achieve low color difference and avoid the generation of pores during the production process.
(3) The present application adopts a combination of microwave drying and baking, improving the surface wrinkles, softness, and powder flying phenomenon of the powder product, enhancing the yield rate and product quality, and increasing user satisfaction with the powdery product.
(4) In the operation step of bottom vibration with a grid, by adjusting the vibration frequency of the vibrator to 100-500 Hz, the present application can assist material flow and reduce pore formation; additionally, the grid can support the material, making it more robust and leveling the bottom of the material.
(5) The preparation process of the present application does not involve the additional use of chemical auxiliaries, reducing preparation costs, meeting the process requirements of green production. Furthermore, the powder product obtained by the present application has strong stability and is suitable for large-scale industrial production.

### BRIEF DESCRIPTION OF DRAWINGS

Figure 1 is a comparison diagram of the edges of products obtained in Example 1 (right) and Comparative Example 1 (left).
Figure 2 is a comparison diagram of the color difference of products obtained in Example 1 (right) and Comparative Example 2 (left).
Figure 3 is a comparison diagram of the pores of products obtained in Example 1 (right) and Comparative Example 3 (left).
Figure 4 is a comparison diagram of the wrinkles of products obtained in Example 1 (right) and Comparative Example 4 (left).

### DETAILED DESCRIPTION OF THE EMBODIMENTS

### Example 1

The first aspect of this example provided a process method for improving the quality and stability of powder product, comprising the following steps:
S1, Preparation stage: preparing and placing molds;
S2, Preheating a mold: loading the material into the mold, placing the mold in a heating device, and preheating the mold at 58°C;
S3, Filling: placing the material obtained from step S2 into a quantitative filling machine for quantitative filling at a filling temperature of 80°C. According to product weight requirements, the material was quantitatively filled using the quantitative filling machine, with an accuracy of ≤ ±0.3g;
S4, Bottom vibration with a grid: placing the material obtained from step S3 onto a grid and then placing it in a vibrator, vibrating at 200 Hz;
S6, Microwave drying: total microwave power 25 KW: 15-50% duty cycle, microwave frequency: 500 MHz, electromagnetic wave wavelength: 0.05 meters, line speed: 0.5 m/min, temperature inside the tunnel chamber: 30°C;
S7, Pre-freezing: subjecting the material obtained from step S6 to low-temperature pre-freezing in a freezer at -50°C for 5 min;
S8, Demolding: placing the material obtained from step S7 into a demolding machine for demolding;
S9, Freezing: placing the material obtained from step S8 into a freeze dryer, freeze-drying at -45°C for 0.5 h, for re-shaping;
S10, Sublimation drying: placing the material obtained from step S9 into a temperature-programmed freeze dryer for stepped heating drying, negative pressure: -0.9 MPa, increments of 10°C per step, each step lasting 2 h, for a total duration of 12 h;
S11, Baking: baking the material obtained from step S10 in a drying room at 50°C for 8 h;
S12, Storage: storing the material obtained from step S11 in a constant temperature and humidity environment to obtain the powder pre-finished product.

The second aspect of this example provided use of the process method for improving the quality and stability of powder product, specifically applied in the preparation of powder cosmetics. The raw materials for preparing the powder cosmetics comprised an oil phase, an aqueous phase, and a powder phase, wherein the mass ratio of the oil phase, the aqueous phase, and the powder phase was 10:50:40. The oil phase comprised Tween 60 as emulsifier, octyldodecyl stearoyloxy stearate, cetyl ethylhexanoate as thickener, and squalane as humectant, wherein the mass ratio of Tween 60, cetyl ethylhexanoate, octyldodecyl stearoyloxy stearate, and squalane was 2:5:2:2. The aqueous phase comprised deionized water as solvent, carrageenan as thickener, potassium chloride, and caprylyl glycol/ethylhexylglycerin as preservatives, wherein the mass ratio of deionized water, carrageenan, potassium chloride, and caprylyl glycol/ethylhexylglycerin was 0.3:0.1:0.6:99. The powder phase comprised lauroyl lysine as humectant, alumina as filler, silica, boron nitride, and synthetic fluorphlogopite, wherein the mass ratio of lauroyl lysine, alumina, silica, boron nitride, and synthetic fluorphlogopite was 1:1:3:1:30.

### Example 2

The first aspect of this example provided a process method for improving the quality and stability of powder product, comprising the following steps:
S1, Preparation stage: preparing and placing molds;
S2, Preheating a mold: loading the material into the mold, placing the mold in a heating device, and preheating the mold at 60°C;
S3, Filling: placing the material obtained from step S2 into a quantitative filling machine for quantitative filling at a filling temperature of 75°C. According to product weight requirements, the material was quantitatively filled using the quantitative filling machine, with an accuracy of ≤±0.3g;
S4, Bottom vibration with a grid: placing the material obtained from step S3 onto a grid and then placing it in a vibrator, vibrating at 400 Hz;
S6, Microwave drying: total microwave power 25 KW: 15-50% duty cycle, microwave frequency: 100 GHz, electromagnetic wave wavelength: 0.2 meters, line speed: 1.0 m/min, temperature inside the tunnel chamber: 60°C;
S7, Pre-freezing: subjecting the material obtained from step S6 to low-temperature pre-freezing in a freezer at -70°C for 3 min;
S8, Demolding: placing the material obtained from step S7 into a demolding machine for demolding;
S9, Freezing: placing the material obtained from step S8 into a freeze dryer, freeze-drying at -45°C for 0.5 h, for re-shaping;
S10, Sublimation drying: placing the material obtained from step S9 into a temperature-programmed freeze dryer for stepped heating drying, negative pressure: -0.4 MPa, increments of 10°C per step, each step lasting 2 h, for a total duration of 12 h;
S11, Baking: baking the material obtained from step S10 in a drying room at 50°C for 8 h;
S12, Storage: storing the material obtained from step S11 in a constant temperature and humidity environment to obtain the powdery pre-finished product.

The second aspect of this example provided use of the process method for improving the quality and stability of powder product, specifically applied in the preparation of powder cosmetics. The raw materials for preparing the powder cosmetics comprised an oil phase, an aqueous phase, and a powder phase, wherein the mass ratio of the oil phase, the aqueous phase, and the powder phase was 10:50:40. The oil phase comprised Tween 60 as emulsifier, octyldodecyl stearoyloxy stearate, cetyl ethylhexanoate as thickener, and squalane as humectant, wherein the mass ratio of Tween 60, cetyl ethylhexanoate, octyldodecyl stearoyloxy stearate, and squalane was 2:5:2:2. The aqueous phase comprised deionized water as solvent, carrageenan as thickener, potassium chloride, and caprylyl glycol/ethylhexylglycerin as preservatives, wherein the mass ratio of deionized water, carrageenan, potassium chloride, and caprylyl glycol/ethylhexylglycerin was 0.3:0.1:0.6:99. The powder phase comprised lauroyl lysine as humectant, alumina as filler, silica, boron nitride, and synthetic fluorphlogopite, wherein the mass ratio of lauroyl lysine, alumina, silica, boron nitride, and synthetic fluorphlogopite was 1:1:3:1:30.

### Example 3

The first aspect of this example provided a process method for improving the quality and stability of powder product, comprising the following steps:
S1, Preparation stage: preparing and placing molds;
S2, Preheating a mold: loading the material into the mold, placing the mold in a heating device, and preheating the mold at 70°C;
S3, Filling: placing the material obtained from step S2 into a quantitative filling machine for quantitative filling at a filling temperature of 90°C. According to product weight requirements, the material was quantitatively filled using the quantitative filling machine, with an accuracy of ≤±0.3g;
S4, Bottom vibration with a grid: placing the material obtained from step S3 onto a grid and then placing it in a vibrator, vibrating at 500 Hz;
S6, Microwave drying: total microwave power 25 KW: 15-50% duty cycle, microwave frequency: 1000 MHz, electromagnetic wave wavelength: 0.03 meters, line speed: 2.0 m/min, temperature inside the tunnel chamber: 38°C;
S7, Pre-freezing: subjecting the material obtained from step S6 to low-temperature pre-freezing in a freezer at -60°C for 3 min;
S8, Demolding: placing the material obtained from step S7 into a demolding machine for demolding;
S9, Freezing: placing the material obtained from step S8 into a freeze dryer, freeze-drying at -45°C for 0.5 h, for re-shaping;
S10, Sublimation drying: placing the material obtained from step S9 into a temperature-programmed freeze dryer for stepped heating drying: negative pressure: -0.8 MPa, increments of 10°C per step, each step lasting 2 h, for a total duration of 12 h;
S11, Baking: baking the material obtained from step S10 in a drying room at 50°C for 8 h;
S12, Storage: storing the material obtained from step S11 in a constant temperature and humidity environment to obtain the powdery product pre-finished product.

The second aspect of this example provided use of the process method for improving the quality and stability of powder product, specifically applied in the preparation of powder cosmetics. The raw materials for preparing the powder cosmetics comprised an oil phase, an aqueous phase, and a powder phase, wherein the mass ratio of the oil phase, the aqueous phase, and the powder phase was 10:50:40. The oil phase comprised Tween 60 as emulsifier, octyldodecyl stearoyloxy stearate, cetyl ethylhexanoate as thickener, and squalane as humectant, wherein the mass ratio of Tween 60, cetyl ethylhexanoate, octyldodecyl stearoyloxy stearate, and squalane was 2:5:2:2. The aqueous phase comprised deionized water as solvent, carrageenan as thickener, potassium chloride, and caprylyl glycol/ethylhexylglycerin as preservatives, wherein the mass ratio of deionized water, carrageenan, potassium chloride, and caprylyl glycol/ethylhexylglycerin was 0.3:0.1:0.6:99. The powder phase comprised lauroyl lysine as humectant, alumina as filler, silica, boron nitride, and synthetic fluorphlogopite, wherein the mass ratio of lauroyl lysine, alumina, silica, boron nitride, and synthetic fluorphlogopite was 1:1:3:1:30.

### Comparative Example 1

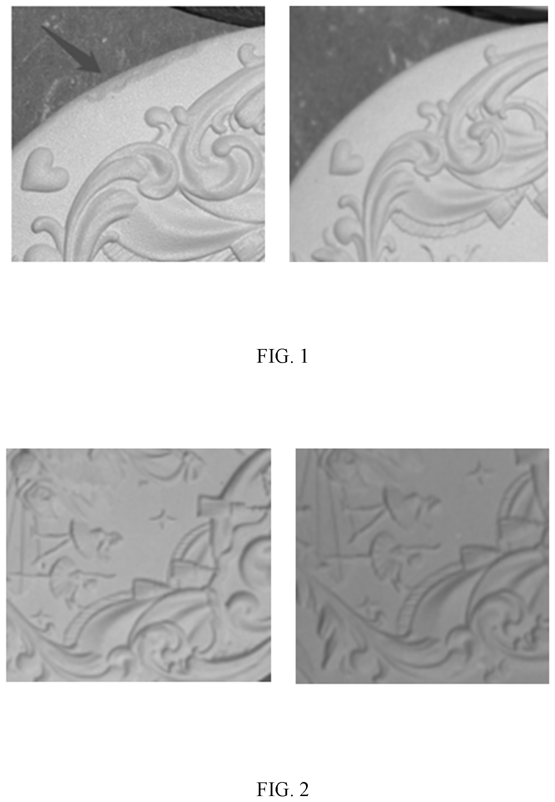

The specific implementation of Comparative Example 1 was the same as Example 1, except that the vibration frequency in step S4 was 800 Hz. The obtained powder product was shown in Figure 1.

A comparison diagram of the edges of products obtained in Example 1 (right) and Comparative Example 1 (left) was shown in Figure 1.

### Comparative Example 2

The specific implementation of Comparative Example 2 was the same as Example 1, except that the mold preheating temperature in step S2 was 100°C. The obtained powder product was shown in Figure 2.

A comparison diagram of the color difference of products obtained in Example 1 (right) and Comparative Example 2 (left) was shown in Figure 2.

### Comparative Example 3

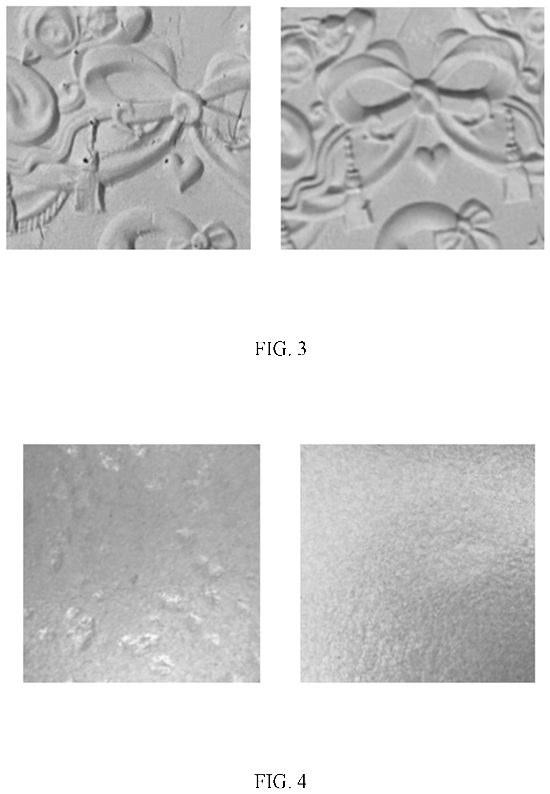

The specific implementation of Comparative Example 3 was the same as Example 1, except that step S2 was absent. The obtained powder product was shown in Figure 3.

A comparison diagram of the pores of products obtained in Example 1 (right) and Comparative Example 3 (left) was shown in Figure 3.

### Comparative Example 4

The specific implementation of Comparative Example 4 was the same as Example 1, except that step S11 was absent. The obtained powder product was shown in Figure 4.

A comparison diagram of the wrinkles of products obtained in Example 1 (right) and Comparative Example 4 (left) was shown in Figure 4.

### Performance Evaluation

Test subjects: The processes of Examples 1-3 and Comparative Examples 1-4 were applied to the powder product formulation provided in Example 1, and the obtained powder products were analyzed and compared.

Test method: Visually observe the appearance of the corresponding powder finished products for edge defects, color difference, pores, and wrinkles on the surface, and record the test results in Table 1.

**Table 1**

| No. | **Appearance** |
|---|---|
| Example 1 | No edge defects, low surface color difference, no pores or wrinkles |
| Example 2 | No edge defects, low surface color difference, no pores or wrinkles |
| Example 3 | No edge defects, low surface color difference, no pores or wrinkles |
| Comparative Example 1 | Phenomenon of not reaching the edge observed |
| Comparative Example 2 | Obvious color difference observed |
| Comparative Example 3 | Multiple pores observed |
| Comparative Example 4 | Obvious wrinkles observed |

## Claims

1. A process method for improving the quality and stability of powder product, **characterized in that**, the process comprises at least the following steps:
preparation stage: preparing and arranging a mold;
preheating a mold: loading the material into the mold, placing the mold into a heating equipment to preheat the mold;
filling: placing the material obtained from the preheating treatment into a quantitative filling machine for quantitative filling, filling the material quantitatively according to the product weight requirement, with an accuracy of ≤ ±0.3g;
bottom vibration with a grid: placing the material obtained from the filling treatment onto a grid and then placing it in a vibrator to vibrate at a certain frequency;
cooling: cooling the material obtained from the vibration treatment in a low-temperature environment;
microwave drying: performing microwave drying on the material obtained from the low-temperature cooling;
pre-freezing: performing pre-freezing on the material obtained from the microwave drying treatment;
demolding: demolding the material obtained from the pre-freezing treatment;
freezing: placing the material obtained from the demolding treatment into a freeze dryer for freezing and shaping;
sublimation drying: placing the material obtained from the freezing and shaping treatment into a freeze dryer for stepwise sublimation drying;
baking: baking the material obtained from the sublimation drying treatment;
storage: storing the material obtained from the baking treatment in a constant temperature and humidity environment to obtain the final product.

2. The process method for improving the quality and stability of powder product according to claim 1, **characterized in that**, a temperature in the step for preheating the mold is 50-350°C.

3. The process method for improving the quality and stability of powder product according to claim 1, **characterized in that**, a vibration frequency of the vibrator in the bottom vibration step with a grid is 100-500Hz.

4. The process method for improving the quality and stability of powder product according to claim 1, **characterized in that**, the equipment used in the cooling step comprises one of a freezing tunnel, a freezing air hood, and a freezing machine.

5. The process method for improving the quality and stability of powder product according to claim 1, **characterized in that**, the operating conditions of the cooling step are as follows: the low-temperature environment is from -10°C to 25°C, and the cooling time is 2-30min.

6. The process method for improving the quality and stability of powder product according to claim 1, **characterized in that**, the microwave drying step is microwave drying; preferably, the conditions of the microwave drying are as follows: total microwave power 25KW: 15-50% duty cycle, microwave frequency: 300MHz-300GHz, electromagnetic waves with a wavelength of 1 meter to 1 millimeter, line speed: 0.5-2m/min, temperature inside the tunnel cavity: 25-65°C.

7. The process method for improving the quality and stability of powder product according to claim 1, **characterized in that**, the freeze-drying equipment in the pre-freezing step comprises one of a liquid nitrogen tunnel, a freezing tunnel, and a freeze dryer, the freeze-drying temperature is from -10°C to 70°C, and the freeze-drying time is 0.1-2h.

8. The process method for improving the quality and stability of powder product according to claim 1, **characterized in that**, the stepwise heating in the sublimation drying step is performed in increments of 5-15°C per step, with each step lasting 1-4 h, for a total duration of 12-24 h.

9. Use of the process method for improving the quality and stability of powder product according to any one of claims 1-8, **characterized in that**, it is applied in the preparation of powder cosmetics.

10. The use of the process method for improving the quality and stability of powder product according to claim 9, **characterized in that**, the raw materials for preparing the powder cosmetics comprise an oil phase, an aqueous phase, and a powder phase; the aqueous phase comprises one or more of a solution, a thickener, an emulsifier, and a preservative; the oil phase comprises one or more of a moisturizer, an emulsifier, a thickener, a fragrance, and a functional component; the powder phase comprises one or more of a filler, a moisturizer, an antioxidant, and a fragrance.
